(19) Europäisches Patentamt
European Patent Office
Office européen des brevets

(11) Publication number: **0 166 089**
**B1**

(12) # EUROPEAN PATENT SPECIFICATION

(45) Date of publication of patent specification: **15.03.89**

(51) Int. Cl.$^4$: **C 07 H 15/04**, C 07 H 15/26, C 08 B 37/00, C 08 B 31/12, C 08 G 81/02

(21) Application number: **85103912.3**

(22) Date of filing: **01.04.85**

(54) **Novel monomeric cationic glycoside derivatives.**

(30) Priority: **27.06.84 US 625289**

(43) Date of publication of application:
**02.01.86 Bulletin 86/01**

(45) Publication of the grant of the patent:
**15.03.89 Bulletin 89/11**

(84) Designated Contracting States:
**AT BE CH DE FR GB IT LI LU NL SE**

(56) References cited:
**US-A-3 321 649**
**US-A-3 669 955**
**US-A-3 931 148**
**US-A-4 469 873**

(73) Proprietor: **National Starch and Chemical Corporation**
**Box 6500**
**Bridgewater, N.J. 08807 (US)**

(72) Inventor: **Tsai, John Ji-Hsiung**
**95 Royce Brook Road**
**Belle Mead New Jersey 08502 (US)**
Inventor: **Tessler, Martin**
**507 Darwin Blvd.**
**Edison New Jersey 08820 (US)**

(74) Representative: **Hagemann, Heinrich, Dr. Dipl.-Chem. et al**
**Patentanwälte HAGEMANN & KEHL Ismaninger Strasse 108 Postfach 860329**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 166 089 B1**

Courier Press, Leamington Spa, England.

## Description

This invention relates to cationic unsaturated mono- and polysaccharide glycoside derivatives, as well as to the polymers derived therefrom.

Ethylenically unsaturated mono- and disaccharide glycoside derivatives as well as homo- and copolymers derived therefrom have been prepared. See, for example, U.S. Pat. No. 3,356,652 issued on December 5, 1967 to D. Ray-Chaudhuri which describes the preparation of a glycoside containing an ethylenically unsaturated side-chain linked to the number 1 carbon of a 2,3,4,6-tetra-O-acetylglucose molecule by reacting a tetraacetylglycosyl halide with a monohydroxy or monocarboxy ethylenically unsaturated monomer. The homo- and copolymers prepared from the acetylated glycosidic monomers are soluble in organic solvents. Upon deactylation, the homo- and copolymers having a mole fraction of at least about 20% of the glucoside derivatives become readily water soluble with a greater hydrophilic character than other commonly available synthetic water soluble polymers. The polymers are described as having broad utility in the adhesive, textile, and paper industries.

Similarly, U.S. Pat. No. 4,328,337 issued May 4, 1982 to T. Kawasaki et al. describes the preparation of high polymeric substances having repeating mono- or disaccharide side chains prepared by homopolymerizing (meth)acroyl mono- or disaccharide glycosides. These high polymers are described as being water soluble with excellent bio-adaptability and having a membrane-forming property. When crosslinked, the homopolymers have a high water-retaining property which is useful for many medical treatments.

In U.S. Pat. No. 3,931,148 issued on January 6, 1976 to W. Langdon, novel neutral and cationic glycosidic surfactants are prepared by reacting a 2-hydroxy-3-chloropropyl glycoside of a mono- or polysaccharide with an alkyl amine which contains at least one hydrophobic $C_8$—$C_{18}$ alkyl group. The alkyl amines are described as having 8—30 carbon atoms which may be primary, secondary, tertiary, aliphatic, saturated or unsaturated, alicyclic and aralkyl. The glycosides are described as being useful in areas requiring surfactants exhibiting biodegradability, alkali solubility and stability.

In US—A—3 669 955, cationic polyol derivatives are prepared inter alia by reaction of 3-halo-2-hydroxypropyl pyridinium salts with a polyol such as starch. These compounds are described as being useful in flocculating applications and in paper treating and coating.

Due to the low cost and abundance of many saccharides in addition to the hydrophilic nature they provide to polymers, it is the prime objective herein to produce a novel class of cationic unsaturated mono- and polysaccharide glycoside derivatives, the derivatives being capable of undergoing homopolymerization or copolymerization in the presence of other unsaturated comonomers. None of the above references disclose or suggest the products of the present invention.

Novel cationic unsaturated mono- and polysaccharide glycoside derivatives are provided which have the following structure:

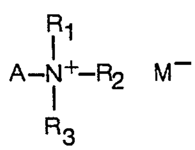 or 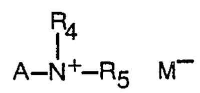

wherein A is

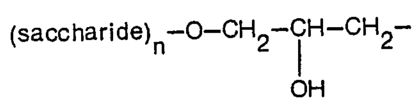

and (saccharide)$_n$—O— represents a mono- or polysaccharide where O is attached to the glycosidic carbon atom in the terminal saccharide ring of (saccharide)$_n$ and n is 1 to 20;
wherein

$R_1$ may be H; $C_1$—$C_6$ alkyl; $C_2$—$C_6$ alkenyl or alkynyl; benzyl; or A;

$R_2$ may be H, $C_1$—$C_6$ alkyl; $C_2$—$C_6$ alkenyl or alkynyl; benzyl; or A;

$R_3$ may be $C_2$—$C_7$ alkenyl or alkynyl or represented by the formula —Z—C[Y]=$CH_2$ or —Z—C≡CH wherein Z is a divalent organo group containing a polar activating group and Y is H or $C_1$—$C_3$ alkyl; or wherein $R_2$ and $R_3$ together with the nitrogen atom to which they are bonded may form optionally a 5 or 6 member saturated heterocyclic ring substituted with a group represented by the formula —C[Y]=$CH_2$ or —C≡CH; wherein $R_4$ and $R_5$ together with the nitrogen atom to which they are bonded optionally form a 5 or 6 member unsaturated heterocyclic ring substituted with a group represented by the formula —C[Y]=$CH_2$ or —C≡CH; and wherein M is an anion.

The derivatives may be homopolymerized or copolymerized to produce novel cationic polymers having pendant saccharide side chains which are useful as flocculants and in papermaking.

Glycosides may be prepared from mono- and polysaccharides which contain a reducing carbon atom. This carbon atom, which is located in the terminal saccharide ring, is capable of reacting with alcohol to

form glycosidic products attached by an acetal or ketal linkage, depending on the mono- or polysaccharide employed.

The glycosides which are applicable for use as intermediates in preparing the novel monomeric derivatives herein include halohydrin or glycidyl glycosides having the general formula:

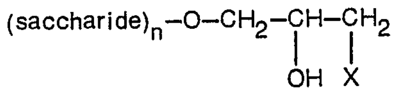

or

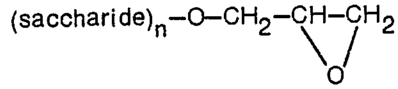

wherein (saccharide)$_n$—O— represents a mono- or polysaccharide where O is attached to the glycosidic carbon atom in the terminal saccharide ring of (saccharide)$_n$, X is chlorine or bromine, and n is 1 to 20.

W. Langdon (discussed above) prepares similar glycosides by reacting monosaccharides and polysaccharides which are hydrolysable to monosaccharides (including starch and cellulose) at temperatures of about 94 to 108°C. with 3-chloro-1,2-propandiol in the presence of about 0.01 to 2.0 weight percent, based on the reactants, of a strong acid catalyst. The procedure produces severely hydrolyzed products in poor yields which are a dark color, probably due to charring caused by the acid at such high reaction temperatures.

Herein, the glycosides are preferably prepared by reacting a mono- or polysaccharide in an excess of 3-halo-1,2-propandiol in the presence of a cationic exchange resin. By employing a cationic exchange resin, mono- and polysaccharide glycosides may be prepared at moderate temperatures without charring and with only minimal degradation occurring. Additionally, no neutralization step is required as in acid catalyzed systems as the catalyst may be easily removed by filtration.

The reaction is conducted with stirring at a temperature of 55—80°C., preferably 60—65°C over a period of 3—20 hours, preferably 6—8 hours. By employing the preferred lower temperatures and shortened reaction times, the amount of oligosaccharide formation and polysaccharide degradation is reduced. After the reaction is complete, the mixture is filtered in order to remove the cationic exchange resin. The excess diol may then be removed by a number of methods including, for example, vacuum distillation or washing with organic solvents in order to obtain the 3-halo-2-hydroxypropyl glycoside. When monosaccharide glycoside reagents are prepared, the diol may be removed from the glycoside by vacuum distillation, preferably at a temperature of about 80°C. and a pressure of 2 mm Hg. or lower temperatures and pressures. After distillation, the glycoside may optionally be washed with an organic solvent such as acetone or ethyl acetate. Glycosides prepared with polysaccharides may be purified by vacuum distillation, however, distillation temperatures above about 60°C. may cause some degradation. These glycosides are preferably recovered by suspending the glycoside/diol mixture in an organic solvent and filtering a number of times to remove the excess diol and other impurities.

The glycidyl glycosides useful herein may be prepared by reacting a 3-halo-2-hydroxypropyl glycoside with an alkali metal hydroxide in order to form the epoxide group. Typically, the glycoside is mixed with an aqueous alkaline solution while cooling. The mixture is neutralized with acid and then dissolved in alcohol in order to precipitate the metal salts formed. After filtration, the glycidyl glycoside may be recovered by removing the alcohol and water by vacuum distillation.

The monosaccharides which may be employed in the preparation of the glycoside reagent include glycose, fructose, sorbose, mannose, galactose, talose, allose, altrose, gulose, idose, arabinose, xylose, lyxose, ribose, and other similar monosaccharides. Polysaccharides which may be employed in the preparation of the glycosides include maltose, gentiobiose, lactose, cellobiose, maltodextrins of starch having a dextrose equivalent (D.E.) of 5 or greater and other similar polysaccharides comprising no more than about 20 saccharide units.

The halogenated propandiols which may be employed include 3-chloro-1,2-propandiol and 3-bromo-1,2-propandiol. The use of the chloro derivative is preferred due to its commercial availability and cost. The particular saccharide employed and its degree of solubility in the halogenated propandiol will determine the minimum amount of reagent required. While a saccharide to diol ratio of as little as 1:1.4 has been employed, a preferred ratio is at least 1:3 to 1:6, most preferably 1:5. As described above, monosaccharides and polysaccharides of up to about 20 saccharide units which contain a reducing carbon atom are applicable herein. It was found that as the number of saccharide units increases the polysaccharide becomes less reactive and more difficult to dissolve in the 3-halo-1,2-propandiol without employing undesirably high temperatures which cause significant degradation.

Any cationic exchange resin may be used in the glycoside preparation. Suitable exchange resins include sulfonated-crosslinked polystyrene such as commercially available Amberlite IR—120 from Rohm and Haas, Dowex 50 from Dow Chemical and Permutit Q from Permutit; sulfonated phenolics such as Duolite C—3 from Diamond Shamrock; and sulfonated coals such as Zeo Karb H from Permutit. The preferred cationic exchange resin is Dowex 50. The amount of resin useful herein is about 1 part resin of 2—8 parts by weight of saccharide, preferably 1 part resin to 4—5 parts saccharide.

In the process of preparing the novel monomeric cationic glycoside derivatives, the halohydrin or glycidyl glycoside is reacted with an unsaturated amine represented by the formula:

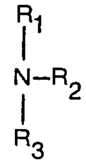

or

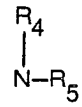

wherein

$R_1$ may be H; $C_1$—$C_6$ alkyl; $C_2$—$C_6$ alkenyl or alkynyl; or benzyl;

$R_2$ may be H, $C_1$—$C_6$ alkyl; $C_2$—$C_6$ alkenyl or alkynyl; or benzyl;

$R_3$ may be $C_2$—$C_7$ alkenyl or alkynyl or represented by the formula —Z—C[Y]=$CH_2$ or —Z—C≡CH wherein Z is a divalent organo group containing a polar activating group and Y is H or $C_1$—$C_3$ alkyl; or wherein $R_2$ and $R_3$ together with the nitrogen atom to which they are bonded may form optionally a 5 or 6 member saturated heterocyclic ring substituted with a group represented by the formula —C[Y]=$CH_2$ or —C≡CH; and wherein $R_4$ and $R_5$ together with the nitrogen atom to which they are bonded optionally form a 5 or 6 member unsaturated heterocyclic ring substituted with a group represented by the formula —C[Y]=$CH_2$ or —C≡CH. The heterocyclic rings will generally comprise at least two carbon atoms with the remaining ring constituents selected from such atoms, for example, as oxygen, nitrogen, sulfur, and phosphorus. Among the amines which are applicable for use in this process, one may list, for example:

N,N-dimethylaminopropyl (meth)acrylamide
N,N-diethylaminopropyl (meth)acrylamide
N,N-dipropylaminopropyl (meth)acrylamide
N,N-dimethylaminopropyl (meth)acrylate
N,N-diethylaminopropyl (meth)acrylate
N,N-dipropylaminopropyl (meth)acrylate
N,N-dimethylaminoethyl (methyl)acrylate
N,N-dipropylaminoethyl (methyl)acrylate
N,N-dibutylaminoethyl (methyl)acrylate
N,N-diisopropylaminoethyl (methyl)acrylate
N-methyl diallyl amine
triallyl amine
vinyl pyridine
vinyl pyrrole
vinyl imidazole
N-methyl vinyl pyrrolidine
N-methyl vinyl piperidine
N-methyl vinyl piperazine

The stearic configuration, polarity, and the position of the unsaturation will affect the polymerizability of the novel cationic glycoside monomers produced. Monomers containing polar groups juxtapositional to the unsaturated bond which active the bond in the presence of free-radical initiating systems will polymerize more readily than monomers which do not contain such polar groups. Monomers containing polar groups which are not juxtapositional to the unsaturated bond and as such will not activate the bond are also not as reactive. Illustrative activating polar groups include

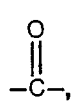, 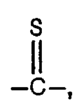, 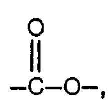, 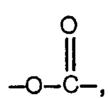, 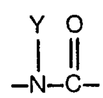

groups and the like. Amines represented by the formula

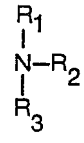

wherein $R_1$ and $R_2$ are described above and $R_3$ is —Z—C[Y]=$CH_2$ wherein Y is described above and Z represents a divalent organo group which contains a polar group juxtapositional to the unsaturated group are preferably employed in the preparation of the novel glycoside monomers.

Either the halohydrin or glycidyl glycoside reagent may be used in the preparation of the novel monomers herein as the glycosides will only react with the amines under alkaline conditions after the halohydrin is first converted to the epoxide form. It should be noted that the amines employed will generally provide sufficient alkalinity to allow the reaction to proceed without the need for additional base.

For this reason, the use of the halohydrin glycoside reagent is preferred.

The unsaturated amine selected and the final glycosidic monomer desired will determine the necessary glycoside to amine molar ratio to be employed. When reacting an amine with the glycosidic reagent, generally an equimolar concentration or a concentration of amine amounting to a slight stoichiometric excess in the order of about 10 to 20% of the glycoside is employed. When reacting with a primary amine, one, two or more molar equivalents of glycoside may be employed. It should be understood that stearic hindrance may be a factor as to whether di- or triglycoside monomers may be produced.

In conducting the process herein, the selected unsaturated amine is first slowly added to an aqueous solution of the glycoside reagent. During the addition, the reaction mixture is cooled in order to maintain a temperature below about 60°C. The reaction is then conducted, ordinarily in the presence of a polymerization inhibitor, with stirring at a temperature of 25 to 65°C., preferably 35—42°C. over a period of 6 to 24 hours, preferably 12 to 16 hours. At the higher reaction temperatures, a polymerization inhibitor such as monoethyl ether hydroquinone should be employed. After the reaction is complete, the solution is concentrated by removal of the water by vacuum distillation after which the novel monomer may optionally be washed with an organic solvent such as acetone. While water is the preferred reaction medium of choice, it should be noted that other polar solvents such as, for example, N-methylpyrrolidinone may be employed.

In general, the novel monomers of this invention are hygroscopic syrups or tacky solids depending on the saccharide length of the glycoside reagent employed. When employing tertiary or secondary amines, the cationic glycoside monomers prepared by the above described process are produced in or near quantitative yields. In the case of reactions of primary amines with stoichiometric amounts of glycoside reagent the resultant yields of cationic monomer may be less than quantitative.

In utilizing our cationic unsaturated glycoside derivatives in the preparation of homo- and copolymers, there may be employed any of the usual vinyl polymerization methods which are well known to those skilled in the art and which are particularly suited for the homo- or copolymer whose preparation is desired. Thus, such polymers may be prepared by means of free radical initiated processes utilizing bulk, suspension, solution, or emulsion polymerization techniques.

Preferably the polymers are prepared in water-in-oil self-inverting emulsion form by the processes described in U.S. Pat. Nos. 3,284,393 (issued November 8, 1966 to J. Vanderhoff et al.), 4,022,736 (issued May 10, 1977 to J. Schmitt), 4,077,930 (issued March 7, 1978 to S. K. Lim et al.) and 4,363,886 (issued December 14, 1982 to S. Lipowski et al.). In the process, an aqueous solution of the cationic unsaturated glycoside monomer is mixed with rapid agitation with a hydrophobic liquid and water-in-oil emulsifying agents to form an emulsion and then polymerized in the presence of a free radical polymerization catalyst.

The comonomers which may be utilized together with the above described cationic unsaturated glycoside monomers for the preparation of the copolymers herein can be any unsaturated monomer polymerizable therewith such, for example, as styrene; alpha-methyl styrene; the acrylic and methacrylic acid esters of aliphatic alcohols such as methyl, ethyl, propyl, butyl, isobutyl, amyl, hexyl, octyl, lauryl and stearyl alcohols; the dialkylaminoalkyl esters of acrylic and methacrylic acids; acrylic acid, methacrylic acid; isoprene; acrylamide, acrylonitrile; methacrylonitrile; butadiene; vinyl propionate; dibutyl fumarate and maleate; diallyl phthalate; vinylidene chloride; vinyl chloride; vinyl fluoride; vinyl acetate, ethylene; and, propylene, etc. Any of these comonomers may be used either alone or in combination with one another together with one or more of our cationic monomers.

Many cationic polymers are employed as flocculants for aiding the separation of finely divided particles, such for example, as minerals from aqueous suspension. The rate of flocculation is known to behave as a function of the charge number, molecular weight, and molecular structure of the polymer employed. While the novel cationic glycoside monomers herein do not flocculate clay suspensions, many of the cationic homo- and copolymers described herein do provide good to excellent clay flocculation times. In papermaking, the ease with which water drains from stock on a paper machine "wire" and amount of water retained in the wet web as it passes to and through the presses affects both speed of the machine and quality of the paper. Use of the cationic homo- and copolymers as well as the amphoteric copolymers herein as drainage aids was seen to improve drainage rates. The polymers may also find use as pigment or strength retention aids in papermaking. The following test procedures were used to evaluate the homo- and copolymers described herein:

## Clay Flocculation Test

A total of 38 parts Attasorb clay (obtained from Englehard Industries, Inc.) and 3462 parts water are stirred for 16 hours at room temperature. A portion of this clay suspension is added to fill a 1000 ml graduated cylinder then mixed with a plunger three times. A total of 40 ml. of a 0.1% polymer solution is then added to the clay suspension and again plunged three times. The clay flocculation time is recorded as the number of seconds necessary for the 40 ppm polymer treatment to cause the clay to flocculate and settle to the 700 ml mark of the graduated cylinder. A cationic diethylaminoethyl ether corn starch derivative useful as a clay flocculant (described in U.S. Pat. No. 2,813,093 issued on November 12, 1957 to C. Caldwell et al.) which has a clay flocculation time of 70 seconds was used for comparison.

Evaluation as Drainage Aids

Unbleached soft wood kraft pulp at a 1.5% consistency is beaten in a Valley Beater to approximately 600 C.S.F. (Canadian Standard Freeness). The pulp stock is then aged for two days under ambient air temperature (18—24°C). The pulp is diluted with water to obtain a 0.5% consistency then neutralized to pH 7.0 with sulfuric acid. This pulp stock is used for the drainage test in a neutral system. For the drainage test in an acid system, alum (approximately 3.3% on dry pulp basis) is added to the neutralized pulp stock to adjust the pH to 5.5. Samples of the polymers are added at treatment levels of 0.1 to 0.5% on dry pulp basis to a 345 ml aliquot of either the neutral or acid pulp stock. After mixing for one minute, the treated pulp stock is added to a graduated dynamic drainage cylinder containing 1553 ml of 100 ppm $CaCO_3$ water which has a marine type propeller positioned at the 500 ml mark. A predetermined drainage volume of 1200 ml was chosen. After the pulp slurry is mixed for 30 seconds, the stopper at the bottom of the cylinder is pulled. When the pulp stock volume is drained to the 1500 ml mark, the seconds timer is started. After thee volume is drained to the 300 ml cylinder mark, the timer is stopped. A sample's drainage rate is measured in ml/sec with the best drainage aid having the fastest drainage rate.

The control for the drainage test in a neutral system consisted of a cationic starch ether derivative of the prior art, i.e. the diethylaminoethyl ether of waxy maize containing 0.27% nitrogen by weight (dry basis). The control for the acid system consisted of an amphoteric starch ether derivative of the prior art, i.e. the phosphorylated diethylaminoethyl ether of waxy maize containing 0.27% nitrogen and 0.1% phosphorus by weight (dry basis). Both starch derivatives were prepared as described in U.S. Pat. No. 3,459,632 issued on August 5, 1969 to C. G. Caldwell et al.

In the examples which follow, all parts and percentages are given by weight and all temperatures are in degrees Celsius unless otherwise noted.

Example 1

This example illustrates the preparation of 3-chloro-2-hydroxypropyl glucoglycoside.

To a 0.5 liter round-bottom flask equipped with condenser, mechanical stirrer and means for heating, there was added 80 g. (0.44 mole) of dextrose, 237 g. (2.15 moles) of 3-chloro-1,2-propandiol, and 20 g. Dowex 50W—X8 cationic exchange resin (1.9 meq/ml.) in H+ form. The mixture was heated to 60°C and stirred at that temperature for 16 hours. The reaction mixture was cooled and then filtered over a gauze cloth to remove the resin. The reaction mixture was clear and light yellow in color. Unreacted diol was removed by vacuum distillation at 80°C at 2 mm Hg. The hygroscopic solid product was slurried in acetone and filtered three times to remove residual impurities then dried in a vacuum dessicator. The light beige colored glycoside was recovered in an 80% yield (based on theoretical). $C^{13}$ NMR spectral analysis indicated the absence of the reducing carbon atom hemi-acetal signals at 92 and 96 ppm. Signals were recorded indicating a glycosidic carbon at 100.2 and 104.3 ppm corresponding to an acetal linkage. Organic chlorine analysis showed the glycoside to contain 11.5% organic chlorine instead of the expected value of 13.02% based on a 272.54 molecular weight of the glycoside. This indicates that a small degree of oligosaccharide formation occurred resulting in a product containing both the glucoglycoside as well as a small amount of oligosaccharde glycoside.

Example 2

This example illustrates the preparation of the 3-chloro-2-hydroxypropyl glycoside of a maltodextrin containing ten glucose units connected by either 1,4 or 1,6 linkages, referred to as having a D.E. of 10.

The procedure of Example 1 was followed except that the reaction time was reduced to 6 hours and the vacuum distillation step was omitted. The maltodextrin glycoside was recovered in an 84% yield (based on theoretical). The $C^{13}$ NMR spectra of the glycoside product revealed no signals corresponding to the hemi-acetal form of the reducing carbon atom of the maltodextin. Signals were recorded at 98.6, 99.9, and 102.8 ppm corresponding to the α- and β-glycoside carbon linkages of the maltodextrin. Analysis showed the organic chlorine content of the product to be 2.62% as compared to an expected 2.15% based on the molecular weight of the D.E. 10 glycoside. This indicates the presence of some lower molecular weight chlorohydrin glycoside present caused by slight degradation.

Example 3

This example illustrates the preparation of N-[2-hydroxy-3-glucoglycosyl-propyl], N,N-dimethyl, 3-methacrylamidopropylammonium chloride.

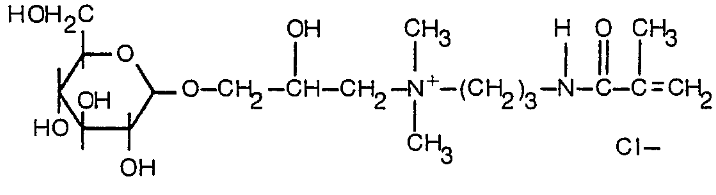

A total of 35.3 g. dry basis (0.13 moles) of 3-chloro-2-hydroxypropyl glucoglycoside of Example 1 was

dissolved in 30 ml. of distilled water in a 250 ml. round bottom flask. While cooling in an ice bath, 22.1 g. (0.13 moles) of N,N-dimethylaminopropyl methacrylamide was added slowly over a period of 30 minutes with an addition funnel. Five drops of a 1% monomethyl ether hydroquinone ethanol solution were added and the reaction mixture was then heated to 40°C with stirring for 16 hours. The mixture was concentrated on a rotary evaporator at 40°C, extracted with acetone to remove any unreacted vinyl monomer, and dried in a vacuum dessicator. The desired product, which will for purposes of brevity be hereinafter referred to as DMAPMA-glucoglycoside (MW ≈ 443), was a yellow syrup.

No organic chlorine was detected by chlorine analysis indicating the reaction was quantitative. IR analysis showed signals at 1605 and 1670 $cm^{-1}$ indicating the presence of the acryloyl functionality. $C^{13}$ NMR spectral analysis showed no reducing glycosidic carbon present but acryloyl carbon atom signals were picked up at 172.4, 139.4, and 122.0 ppm.

Example 4

This example illustrates the preparation of DMAPMA-glucoglycoside employing N-methyl pyrrolidinone as a reaction medium.

Employing the reaction conditions of Example 3, 17.0 g. dry basis (0.062 moles) of 3-chloro-2-hydroxypropyl glucoglycoside of Example 1 was dissolved in 10 ml. of N-methyl pyrrolidinone and reacted with 10.81 g. (0.063 moles) of N,N-dimethylaminopropyl methacrylamide in the presence of 2 drops of 1% monomethyl ether hydroquinone ethanol solution and 8 drops of 2,4,6-trimethylpyridine. The reaction was conducted at a temperature of 60°C for 16 hours and then cooled to room temperature. The DMAPMA-glucoglycoside was recovered by precipitation in acetone and dried in a vacuum dessicator. Based on organic chlorine concentration, the glycoside monomer was prepared in about a 52% yield based on theoretical, indicating that water is a preferred reaction medium for the monomer preparation.

Example 5

Instead of the glucoglycoside, the maltodextrin 10 glycoside of Example 2 was employed in the reaction of Example 3 to obtain a slightly yellow, tacky semi-solid in a quantitative yield based on organic chlorine analysis. According to the results of IR and $C^{13}$ NMR analysis the product was identified as N-[2-hydroxy-3-(maltodextrin 10)-glycosylpropyl], N,N-dimethyl-3-methacrylamidopropylammonium chloride (MW ≈ 1900), hereinafter referred to as DMAPMA-maltodextrin 10-glycoside, having the structure:

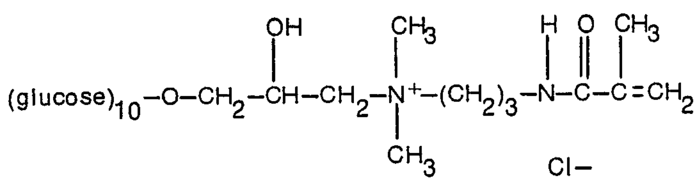

Example 6

This example illustrates the preparation of N-[2-hydroxy-3-glucoglycosyl-propyl]-4-vinylpyridinium chloride.

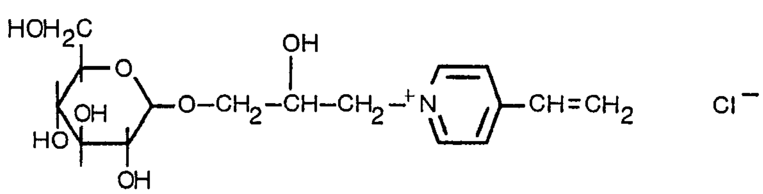

A total of 15 g. dry basis (0.034 moles) of 3-chloro-2-hydroxypropyl glucoglycoside was dissolved in 15 g. of distilled water in a 100 ml. round bottom flask. Six ml. (0.056 moles) of 4-vinyl pyridine was added slowly with cooling. The mixture was heated to 60°C and stirred for 16 hours. The mixture was recovered as in Example 3. The product was a dark red, tacky solid. No organic chlorine was detected indicating the reaction was quantitative. $C^{13}$ NMR analysis showed the presence of the vinyl group at 118.5 and 135.2 ppm, however, the product was shown to contain some polymeric products.

Example 7

This example illustrates the preparation of N-[2-hydroxy-3-glucoglycosyl-propyl], N-methyldiallylammonium chloride.

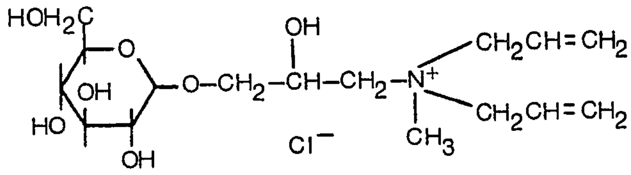

A total of 22.1 g. dry basis (0.08 moles) of 3-chloro-2-hydroxypropyl glucoglycoside was dissolved in 10 g. of water and reacted with 12.0 g. (0.10 moles) of N-methyldiallylamine by the procedure described in Example 3. The monomer, hereinafter referred to as MDAA-glucoglycoside was recovered by precipitation in acetone then dried in a vacuum dessicator. The monomer contained very low levels of organic chlorine indicating that the reaction was almost quantitative.

Example 8

This example illustrates the preparation of N-[2-hydroxy-3-glucoglycosyl-propyl], N-isopropyl, 3-methacrylamidopropylammonium chloride.

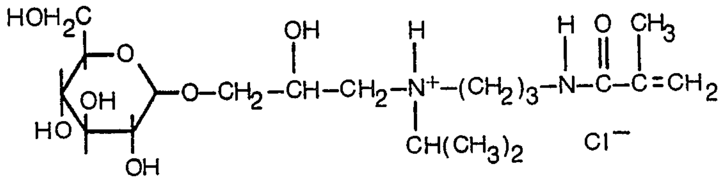

The monomer, referred to as IPAPMA-glucoglycoside was prepared as in Example 6 employing 19.0 g. (0.10 moles) of N-isopropylaminopropyl methacrylamide. This monomer was also recovered in almost a quantitative yield.

Example 9

This example illustrates the preparation of an ethynylically unsaturated cationic glycoside monomer.

A total of 12.4 g. dry basis (0.045 moles) of 3-chloro-2-hydroxypropyl glucoglycoside was dissolved in 15 g. of water and reacted with 2.5 g. (0.045 moles) of propargylamine at 60°C. in the presence of 4 drops of 2,4,6-trimethylpyridine. After 5.5 hours a second equivalent portion of glycoside in water was added with an additional 5.8 g. of 2,4,6-trimethylpyridine. The reaction continued for an additional 15 hours at 60°C. The monomer was recovered by precipitation in acetone in about a 55% yield based on chlorine analysis for a diglycoside product.

Example 10

This example illustrates the solution homopolymerization of DMAPMA-glucoglycoside.

A total of 7.0 g. dry basis of DMAPMA-glucoglycoside and 12.0 g. of degassed water were added to a three-necked round bottom flask equipped with a mechanical stirrer, addition funnel and nitrogen source. Ammonium persulfate (0.070 g. in 4 ml. of water) was added in four increments over a period of two hours. The 30% solids solution was then heated to 70°C and stirred for 6 hours to yield a viscous polymer product. In addition to the observed viscosity of the polymer, polymerization was further verified by the clay flocculation test. The DMAPMA-glucoglycoside did not flocculate clay; however, the cationic homopolymer prepared had a clay flocculation time of 56.5 seconds.

A 79% solids solution containing 1% ammonium persulfate based on the monomer was homopolymerized in a closed vessel under similar conditions to yield a gel with strong water retention properties.

Example 11

This example illustrates the solution homopolymerization of DMAPMA-maltodextrin 10-glycoside.

A total of 4 parts (dry basis) of DMAPMA-maltodextrin 10-glycoside, 6 parts of degassed water and 4 parts 1% ammonium persulfate were added to a vessel and sealed under nitrogen. The solution was heated to 80°C. and allowed to react for 72 hours to yield a viscous polymer solution. A solution of the polymer had a clay flocculation time of 87 seconds.

Example 12

This example illustrates the solution homopolymerizations of MDAA-glucoglycoside and IPAPMA-glucoglycoside.

The glycoside monomers prepared in Examples 7 and 8 were solution homopolymerized as described in Example 11 at 70°C. for 1 hour. Both homopolymers were clear, slightly viscous, and smooth in texture. The MDAA-glucoglycoside homopolymer had a clay flocculation time of 78.5 seconds. The IPAPMA-glucoglycoside homopolymer has a clay flocculation time of 53.8 seconds.

Example 13

This example illustrates the water-in-oil emulsion homopolymerization of DMAPMA-glucoglycoside.

To a 100 ml. three-necked round bottom flask equipped with stirring apparatus, condensor, addition funnel and nitrogen source was added 7.0 g. (dry basis) of DMAPMA-glucoglycoside and 6.0 g. of degassed water. A total of 7.0 g. of Isopar M (branched-chain isoparaffinic oil obtained from Exxon Corporation), and 1.86 g. Tween 85 and 0.47 g. Span 80 (surfactants obtained from Imperial Chemical Industries) were slowly added with rapid stirring to form an emulsion. After the temperature of the emulsion was raised to 70°C a total of 0.02 ml. of Lupersol 11 (a 75% by weight solution of t-butyl peroxypivalate in 0.2 M n-decane obtained from Pennwalt Corporation) which was dissolved in 2 ml. of Isopar M was added in three increments over three hours. The polymerization was continued for an additional three hours and then stopped by the addition of 5 drops of 1% monomethyl ether hydroquinone in ethanol. When inverted in water the polymer had a clay flocculation time of 41 seconds. A 1% solution of the polymer had a Brookfield viscosity of 1000 cps. (Spindle #5 at 20 rpm).

Example 14

This example illustrates the solution copolymerization of DMAPMA-glucoglycoside and acrylamide.

Employing a reaction apparatus similar to that described in Example 7, 6.63 g. dry basis (0.015 moles) of the glycoside monomer and 10.65 g. (0.15 moles) of acrylamide were dissolved in 60 g. of degassed water. After the solution was heated to 65°C, 0.02 g. of 2,2-azobis (2-amidinopropane)hydrochloride in 3 ml. of water was added in three increments over a period of three hours. The reaction was stopped after a total of six hours by the addition of 1% monoethyl ether hydroquinone ethanol solution. The viscous solution of the cationic 1:10 molar ratio DMAPMA-glycoglycoside:acrylamide copolymer was light brown. A similar copolymer was prepared in a 2:10 molar ratio. A mixture of DMAPMA-glycoglycoside and acrylamide did not flocculate clay, however, the solution copolymers prepared had clay flocculation times of 45 to 47 seconds.

Example 15

A 2.8:10 molar ratio solution copolymer of DMAPMA-maltodextrin 10-glycoside:acrylamide was prepared as in Example 11. A viscous solution was obtained after a reaction time of 6 hours. The copolymer had a clay flocculation time of 74 seconds.

Example 16

A solution copolymer of acrylamide and the diglycoside propargyl monomer of Example 9 was prepared as in Example 11 with 4 parts of the cationic monomer (dry basis), 4.5 parts of degassed water, 1 part acrylamide, and 4 parts 1% ammonium persulfate. The reaction was conducted at 75°C for 1 hour resulting in a slightly viscous solution. The solution was cooled to room temperature and then dialyzed in order to recover only copolymers having a molecular weight greater than 1,000. Zeta potential analysis of the copolymer showed it to be cationic, confirming that copolymerization occurred. The polymer had a poor clay flocculation time (>200 seconds) which was probably due to the relatively low molecular weight of the copolymer produced.

Example 17

This example illustrates the preparation of water-in-oil emulsion copolymers and terpolymers of DMAPMA-glucoglycoside, DMAPMA-maltodextrin 10-glycoside, and acrylamide.

Employing an apparatus and procedure similar to that described in Example 13, the comonomers were dissolved in degassed water, formed into an emulsion, then heated to a temperature of 65°C. To each emulsion 0.03 g. of Lupersol 11 in 2 ml. of Isopar M was added in three increments over three hours. Each reaction, complete after 5 hours, was stopped with 5 drops of a 1% monomethy ether hydroquinone ethanol solution. The reaction data is given in Table I.

TABLE I
Copolymerization of
DMAPMA-Glucoglycoside-DMAPMA-Maltodextrin 10-glycoside:Acrylamide

EMULSION PREPARATION

| Polymer | A | B | C | D | E | F |
|---|---|---|---|---|---|---|
| Molar Ratio* | 1:0.10 | 2:0:10 | 1:0.115:10 | 1:0.23:10 | 1:0.47:10 | 0:1:21.45 |
| Ingredients: | | | | | | |
| DMAPMA-glucoglycoside (D.B.) (g.) | 4.64 | 6.96 | 4.64 | 4.64 | 3.71 | 0 |
| DMAPMA-Maltodextrin 10-glycoside (D.B.) (g.) | 0 | 0 | 2.32 | 4.64 | 7.42 | 7.42 |
| Acrylamide (g.) | 7.46 | 5.60 | 7.46 | 7.46 | 7.97 | 5.97 |
| Water (g.) | 15 | 15 | 25 | 25 | 25 | 15 |
| Isopar M (g.) | 16 | 16 | 25 | 25 | 25 | 15 |
| Tween 85 (g.) | 3.72 | 3.72 | 3.72 | 3.72 | 2.97 | 2.97 |
| Span 80 (g.) | 0.94 | 0.94 | 0.92 | 0.92 | 0.74 | 0.74 |
| Data: | | | | | | |
| Clay flocculation (sec.) | 19.0 | 27.0 | 36.0 | 50.2 | 22.6 | 52.7 |
| Brookfield Viscosity (cps.)** | 5,680 | 1,200 | 18,250 | 10,600 | 5,800 | Watery |
| Spindle # | 5 | 4 | 6 | 6 | 5 | — |

* Molar ratio of DMAPMA-glucoglycoside:DMAPMA-maltodextrin 10-glycoside:acrylamide.
** 1% solids at 20 rpm.

It was noted that inverted solutions of the terpolymers containing the maltodextrin 10-glycoside monomer were smooth in contrast to the usual cohesiveness exhibited by cationic copolymers.

Example 18

This example illustrates the preparation of amphoteric terpolymers of DMAPMA-glucoglycoside:acrylamide:acrylic acid having a positive, negative, or neutral overall charge.

Employing an apparatus as described in Example 13, a solution of acrylic acid in degassed water was adjusted to a pH of 4 to 7 with 50% potassium hydroxide with cooling prior to adding the glycoside and acrylamide. The terpolymers were then emulsified and polymerized employing the reaction conditions of Example 16. The reaction data is given in Table II.

Example 19

The emulsion terpolymers G—I described above were evaluated as drainage aids in an acid system. The results are given in Table III.

TABLE III
DRAINAGE IN AN ACID SYSTEM

| Polymer | Drainage Rate (ml./sec.) in Presence of 3.3% Alum at Treatment Levels: 0.1% | 0.2% | % of Control 0.1% | 0.2% |
|---|---|---|---|---|
| Blank | 39.5 | 39.5 | — | — |
| Control* | 62.2 | 88.2 | — | — |
| G | 79.0 | 125.9 | 127.0 | 142.4 |
| H | 82.8 | 107.2 | 133.1 | 121.4 |
| I | 64.5 | 80.3 | 103.8 | 91.0 |
| J | 66.1 | 82.8 | 106.3 | 93.8 |
| K | 43.6 | 47.2 | 70.2 | 53.5 |
| L | 94.5 | 131.2 | 152.0 | 148.0 |

* Phosphorylated diethylaminoethyl ether of waxy maize starch.

TABLE II
Polymerization of DMAPMA-glucoglycoside-Acrylamide:Acrylic Acid

EMULSION PREPARATION

| Polymer | G | H | I | J | K | L | M | N | O |
|---|---|---|---|---|---|---|---|---|---|
| Molar ratio* | 1.25:<br>4.65:<br>1 | 1.25:<br>4.65:<br>1 | 2.5:<br>2.1:<br>1 | 1:4:1 | 0.5:8:3 | 0.5:8:3 | 0.5:9:3 | 0.5:10:4 | 1:10:4 |
| Overall Charge | + | + | + | neutral | – | – | – | – | – |
| pH | 7 | 4 | 4 | 4 | 4 | 4 | 7 | 7 | 7 |
| Ingredients:<br>DMAPMA-gluco-glycoside (D.B.) (g.) | 7.89 | 7.54 | 12.26 | 5.52 | 1.41 | 1.41 | 1.41 | 1.41 | 2.81 |
| Acrylamide (g.) | 4.89 | 4.57 | 1.65 | 3.55 | 3.60 | 3.60 | 4.08 | 4.50 | 4.50 |
| Acrylic Acid (g.) | 1.03 | 0.98 | 0.80 | 0.90 | 1.38 | 1.38 | 1.36 | 1.84 | 1.84 |
| Water (g.) | 14 | 14 | 14 | 10 | 10 | 10 | 10 | 10 | 10 |
| Isopar M (g.) | 16 | 16 | 16 | 7.5 | 8 | 8 | 8 | 8 | 8 |
| Tween 85 (g.) | 3.72 | 3.72 | 3.72 | 0.62 | 0.62 | 2.48 | 2.48 | 2.48 | 2.48 |
| Span 80 (g.) | 0.94 | 0.94 | 0.94 | 2.48 | 2.48 | 0.62 | 0.62 | 0.62 | 0.62 |
| Clay flocculation (sec.) | 26.6 | 28.6 | 35.6 | — | — | — | — | — | — |

* Molar ratio of DMAPMA-glucoglycoside-acrylamide-acrylic acid

The results show that amphoteric terpolymers containing DMAPMA-glucoglycoside were effective drainage aids in an acid system and in many cases performed better than the control.

Example 20

Cationic emulsion polymers were evaluated as drainage aids in a neutral system. The results are given in Table IV.

TABLE IV
DRAINAGE IN A NEUTRAL SYSTEM

| | Drainage Rate (ml./sec.) at Treatment Levels: | | | % of Control | | |
|---|---|---|---|---|---|---|
| Polymer | 0.1% | 0.2% | 0.4% | 0.1% | 0.2% | 0.4% |
| Blank | 32.0 | 32.0 | 32.0 | — | — | — |
| Control* | 38.1 | 49.9 | 74.1 | — | — | — |
| DMAPMA-glucoglycoside homopolymer** | 56.6 | 99.2 | 109.1 | 148.6 | 198.8 | 147.3 |
| A | 59.9 | 101.7 | 127.7 | 157.1 | 203.8 | 172.4 |
| B | 73.2 | 93.0 | 114.8 | 192.1 | 186.4 | 155.0 |
| Blank | 39.3 | 39.2 | — | — | — | — |
| Control* | 38.8 | 50.2 | — | — | — | — |
| G | 37.7 | 47.1 | — | 97.0 | 93.7 | — |
| H | 42.9 | 46.7 | — | 110.4 | 93.0 | |
| I | 43.8 | 59.0 | — | 112.8 | 117.4 | — |

* Diethylaminoethyl ether of waxy maize starch.
** Emulsion homopolymer of Example 13.

The results show that the cationic DMAPMA-glucoglycoside homopolymer and copolymers with acrylamide were better than the control in a neutral drainage system. Amphoteric terpolymers having an overall positive charge which contained the glycoside monomer also performed as well or better than the control.

## Claims

1. As a composition of matter, a cationic glycoside derivative having the structure:

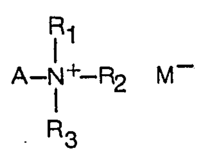
or
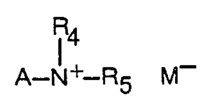

wherein A is

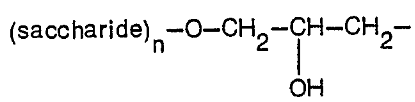

and (saccharide)$_n$—O— represents a mono- or polysaccharide where O is attached to the glycosidic carbon atom in the terminal saccharide ring of (saccharide)$_n$ and n is 1 to 20;
wherein

$R_1$ may be H; $C_1$—$C_6$ alkyl; $C_2$—$C_6$ alkenyl or alkynyl; benzyl; or A;
$R_2$ may be H, $C_1$—$C_6$ alkyl; $C_2$—$C_6$ alkenyl or alkynyl; benzyl; or A;
$R_3$ may be $C_2$—$C_7$ alkenyl or alkynyl or represented by the formula —Z—C[Y]=$CH_2$ or —Z—C≡CH wherein Z is a divalent organo group containing a polar activating group and Y is H or $C_1$—$C_3$ alkyl; or wherein $R_2$ and $R_3$ together with the nitrogen atom to which they are bonded may form a 5 or 6 member saturated heterocyclic ring substituted with a group represented by the formula —C[Y]=$CH_2$ or —C≡CH;

wherein $R_4$ and $R_5$ together with the nitrogen atom to which they are bonded form a 5 or 6 member unsaturated heterocyclic ring substituted with a group represented by the formula $—C[Y]=CH_2$ or $—C{\equiv}CH$; and wherein M is an anion.

2. The glycoside derivative of Claim 1, wherein $R_1$ is H or $C_1—C_6$ alkyl; $R_2$ is H, $C_1—C_6$ alkyl, $C_2—C_6$ alkenyl or alkynyl, or A; $R_3$ is $C_2—C_7$ alkenyl or alkynyl or represented by the formula $—Z—C[Y]=CH_2$ or $—Z—C{\equiv}CH$; or wherein $R_4$ and $R_5$ together with the nitrogen atom to which they are bonded form a 5 or 6 member unsaturated heterocyclic ring substituted with a group represented by the formula $—C[Y]=CH_2$ or $—C{\equiv}CH$.

3. The glycoside derivative of Claim 2, wherein the saccharide unit is glucose, n is 1 and wherein $R_1$ is methyl, $R_2$ is $-CH_2CH=CH_2$, and $R_3$ is $—CH_2CH=CH_2$; $R_1$ is H or A, $R_2$ is H or A, and $R_3$ is $—CH_2C{\equiv}CH$; or $R_4$ and $R_5$ together with the nitrogen form a pyridine ring substituted with $—CH=CH_2$ in a position para to the nitrogen.

4. The glycoside derivative of Claim 2, wherein Z is a divalent organo group containing a polar activating group selected from the group consisting of

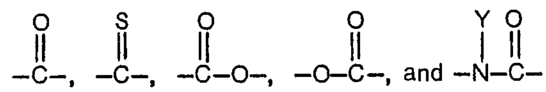

wherein Y is H or $C_1—C_3$ alkyl, the polar activiating group being juxtapositional to the unsaturated group.

5. The glycoside derivative of Claim 4 having the structure:

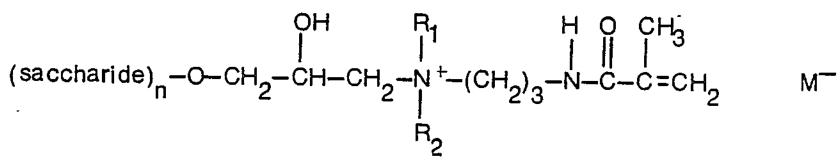

wherein the saccharide unit is glucose, n is 1 to 10, and $R_2$ is H or $C_1—C_6$ alkyl.

6. A process for preparing the cationic glycoside derivative of Claim 1, comprising the steps of:

(a) reacting under alkaline conditions at least one mole of a glycoside reagent having the formula

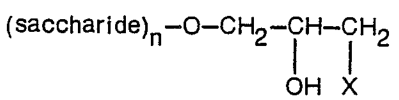

or

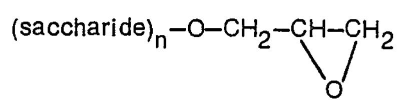

wherein X is chlorine or bromine, with one mole of an unsaturated amine having the formula

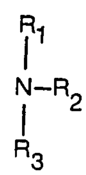

or

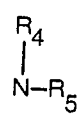

and

b) isolating the resulting cationic glycoside derivative.

7. A polymer of the cationic glycoside derivative of Claim 1, wherein the polymer is a homopolymer or a copolymer with at least one other unsaturated comonomer.

8. The polymer of Claim 7, wherein $R_1$ is H or $C_1—C_6$ alkyl; $R_2$ is H, $C_1—C_6$ alkyl, $C_2—C_6$ alkenyl or alkynyl, or A; $R_3$ is $C_2—C_7$ alkenyl or alkynyl or represented by the formula $—Z—C[Y]=CH_2$ or $—Z—C{\equiv}CH$ wherein Z is a divalent organo group containing a polar activating group situated juxtapositional to the unsaturated group and selected from the group consisting of

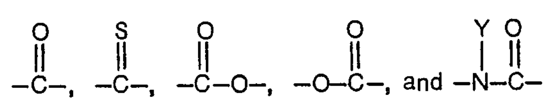

or wherein $R_4$ and $R_5$ together with the nitrogen atom to which they are bonded form a 5 or 6 member unsaturated heterocyclic ring substituted with a group represented by the formula $—C[Y]=CH_2$ or $—C{\equiv}CH$.

9. The polymer of Claim 8, wherein the cationic glycoside monomer has the structure

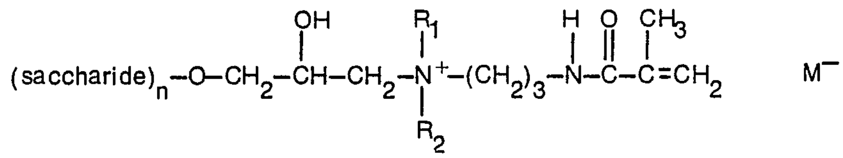

wherein $R_2$ is H or $C_1$—$C_6$ alkyl.

10. The polymer of Claim 7, wherein the unsaturated comonomer is selected from the group consisting of acrylamide, methacrylamide, acrylic acid, and methacrylic acid.

**Patentansprüche**

1. Kationisches Glykosidderivat mit der Struktur

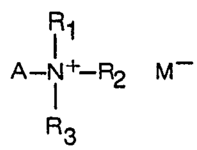

oder

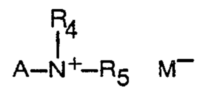

worin A

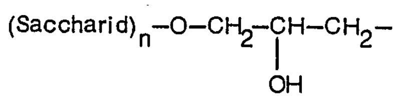

ist und $(Saccharid)_n$—O— ein Mono- oder Polysaccharide bedeutet, worin O an ein glykosidisches Kohlenstoffatom im endständigen Saccharidring von $(Saccharid)_n$ gebunden ist und n 1 bis 20 ist, worin

$R_1$ H, $C_1$—$C_6$-Alkyl, $C_2$—$C_6$-Alkenyl oder -Alkinyl, Benzyl oder A sein kann,

$R_2$ H, $C_1$—$C_6$-Alkyl, $C_2$—$C_6$-Alkenyl oder -Alkinyl, Benzyl oder A sein kann,

$R_3$ $C_2$—$C_7$-Alkenyl oder -Alkinyl sein kann oder durch die Formel —Z—C[Y]=$CH_2$ oder —Z—C≡CH dargestellt wird,

worin Z eine zweiwertige Organogruppe ist, die eine polare, aktivierende Gruppe enthält, und Y H oder $C_1$—$C_3$-Alkyl ist, oder worin $R_2$ und $R_3$ zusammen mit dem Stickstoffatom, an welches sie gebunden sind, gegebenenfalls eine 5- oder 6-gliedrigen, gesättigten, heterozyklischen Ring bilden, der mit einer Gruppe der Formel —C[Y]=$CH_2$ oder —C≡CH substituiert ist, worin $R_4$ und $R_5$ zusammen mit dem Stickstoffatom, an welches sie gebunden sind, einen 5- oder 6-gliedrigen ungesättigten, heterozyklischen Ring bilden, der mit einer Gruppe der Formel —C[Y]=$CH_2$ oder —C≡CH substituiert ist, und worin M ein Anion ist.

2. Glykosidderivat nach Anspruch 1, worin $R_1$ H oder $C_1$—$C_6$-Alkyl ist, $R_2$ H, $C_1$—$C_6$-Alkyl, $C_2$—$C_6$-Alkenyl oder -Alkinyl oder A ist, $R_3$ $C_2$—$C_7$-Alkenyl oder -Alkinyl ist oder durch die Formel —Z—C[Y]=$CH_2$ oder —Z—C≡CH dargestellt wird, oder worin $R_4$ und $R_5$ zusammen mit dem Stickstoffatom, an welches sie gebunden sind, einen 5- oder 6-gliedrigen ungesättigten, heterozyklischen Ring bilden, der mit einer Gruppe der Formel —C[Y]=$CH_2$ oder —C≡CH substituiert ist.

3. Glykosidderivat nach Anspruch 2, worin die Saccharideinheit Glucose ist, n gleich 1 ist und worin $R_1$ mit Methyl ist, $R_2$ —$CH_2CH$=$CH_2$ ist und $R_3$ —$CH_2CH$=$CH_2$ ist, $R_1$ H oder A ist, $R_2$ H oder A ist und $R_3$ —$CH_2C$≡CH, oder $R_4$ und $R_5$ zusammen mit dem Stickstoffatom einen Pyridinring bilden, der in p-Stellung zum Stickstoffatom durch —CH=$CH_2$ substituiert ist.

4. Glykosidderivat nach Anspruch 2, worin Z eine zweiwertige Organogruppe ist, die eine polare, aktivierende Gruppe, ausgewählt aus

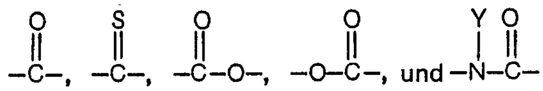

, und

enthält, worin Y H oder $C_1$—$C_3$-Alkyl ist, wobei sich die polare, aktivierende Gruppe neben der ungesättigten Gruppe befindet.

5. Glykosidderivat nach Anspruch 4 mit der Struktur

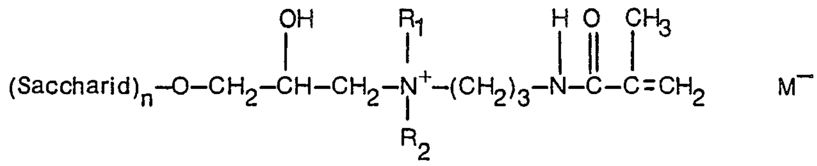

worin die Saccharideinheit Glucose ist, n 1 bis 10 ist und $R_2$ H oder $C_1$—$C_6$-Alkyl ist.

6. Verfahren zur Herstellung des kationischen Glykosidderivats nach Anspruch 1, umfassend die Stufen der

a) Umsetzung von mindestens 1 Mol eines Glykosidreagenzes mit der Formel

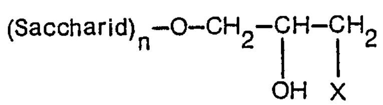

oder

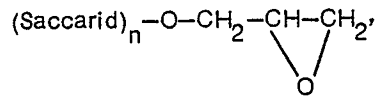

worin X Chlor oder Brom ist, mit 1 Mol eines ungesättigten Amins der Formel

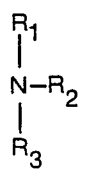

oder

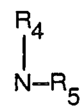

unter alkalischen Bedingungen und

b) Isolieren des erhaltenen kationischen Glykosidderivates.

7. Copolymer des kationischen Glykosidderivats nach Anspruch 1, worin das Polymer ein Homopolymer oder ein Copolymer mit mindestens einem anderen ungesättigten Comonomer ist.

8. Copolymer nach Anspruch 7, worin $R_1$ H oder $C_1$—$C_6$-Alkyl ist, $R_2$ H, $C_1$—$C_6$-Alkyl, $C_2$—$C_6$-Alkenyl oder -Alkinyl oder A ist, $R_3$ $C_2$—$C_7$-Alkenyl oder -Alkinyl ist oder durch die Formel —Z—C[Y]=$CH_3$ oder —Z—C≡CH dargestellt wird, worin Z eine zweiwertige Organogruppe ist, die eine polare, aktivierende Gruppe enthält, die in einer Stellung neben der ungesättigten Gruppe steht und ausgewählt ist aus

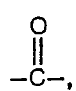,
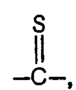,
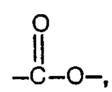,
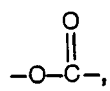, und
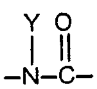

oder worin $R_4$ und $R_5$ zusammen mit dem Stickstoffatom, an welches sie gebunden sind, einen 5- oder 6-gliedrigen ungesättigten, heterozyklischen Ring bilden, der mit einer Gruppe der Formel —C[Y]=$CH_2$ oder —C≡CH substituiert ist.

9. Polymer nach Anspruch 8, worin das kationische Glykosidmonomer die Struktur

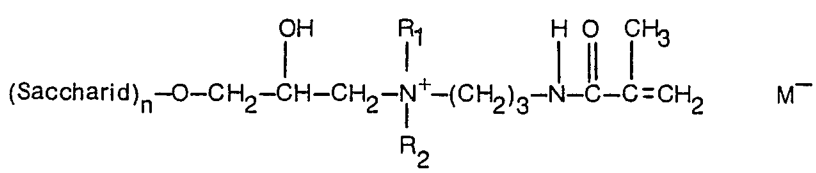

hat, worin $R_2$ H oder $C_1$—$C_6$-Alkyl ist.

10. Polymer nach Anspruch 7, worin das ungestättigte Comonomer ausgewählt ist aus Acrylamid, Methacrylamid, Acrylsäure und Methacrylsäure.

**Revendications**

1. En tant que composition de matière, un dérivé de glucoside cationique ayant la structure suivante:

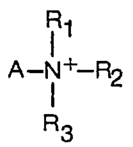 $M^-$ ou 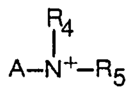 $M^-$

dans laquelle A est

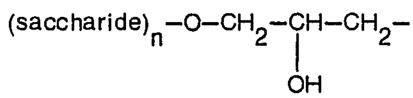

et (saccharide)$_n$—O— représente un mono- ou un polysaccharide, où O est fixé sur l'atome de carbone glucosidique dans le cycle de saccharide terminal de (saccharide)$_n$, et n est compris entre 1 et 20; dans laquelle $R_1$ peut être H, un alcoyle $C_1$—$C_6$, un alcényle ou un alcynyle $C_2$—$C_6$, un benzyle ou A;

$R_2$ peut être H, un alcoyle $C_1$—$C_6$, un alcényle ou un alcynyle $C_2$—$C_6$, un benzyle ou A;

$R_3$ peut être un alcényle ou un alcynyle $C_2$—$C_7$ ou être représenté par la formule:

$$—Z—C[Y]=CH_2 \text{ ou } —Z—C\equiv CH,$$

dans laquelle Z est un groupe organique bivalent contenant un groupe d'activation polaire et Y est H ou un alcoyle $C_1$—$C_3$; ou dans laquelle $R_2$ et $R_3$, ensemble avec l'atome d'azote auquel ils sont liés, peuvent former facultativement un hétérocycle saturé à 5 ou 6 éléments substitué par un groupe représenté par la formule —C[Y]=$CH_2$ ou —C≡CH; dans laquelle $R_4$ et $R_5$, ensemble avec l'atome d'azote auquel ils sont liés, forment facultativement un hétérocycle non saturé à 5 ou 6 éléments substitué par un groupe représenté par la formule —C[Y]=$CH_2$ ou —C≡CH; et dans laquelle M est un anion.

2. Dérivé de glucoside selon la revendication 1, dans lequel:

$R_1$ est H ou un alcoyle $C_1$—$C_6$; $R_2$ est H, un alcoyle $C_1$—$C_6$, un alcényle ou un alcynyle $C_2$—$C_6$, ou A; $R_3$ est un alcényle ou un alcynyle $C_2$—$C_7$ ou est représenté par la formule —Z—C[Y]=$CH_2$ ou —Z—C≡CH; ou dans lequel $R_4$ et $R_5$, ensemble avec l'atome d'azote auquel ils sont liés, forment un hétérocycle non saturé à 5 ou 6 éléments substitués par un groupe représenté par la formule

$$—C[Y]=CH_2 \text{ ou } —C\equiv CH.$$

3. Dérivé de glucoside selon la revendication 2, dans lequel l'unité saccharide est le glucose, n est égal à 1, $R_1$ est le méthyle, $R_2$ est —$CH_2CH$=$CH_2$, et $R_3$ est —$CH_2CH$=$CH_2$; $R_1$ est H ou A, $R_2$ est H ou A et $R_3$ est —$CH_2C$≡$CH_2$; ou $R_4$ et $R_5$, ensemble avec l'azote, forment un cycle pyridine substitué par —CH=$CH_2$ en position para par rapport à l'azote.

4. Dérivé de glucoside selon la revendication 2, dans lequel Z est un groupe organique bivalent contenant un groupe d'activation polaire choisi dans le groupe comprenant:

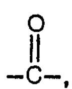, 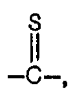, 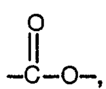, 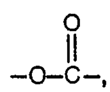, et 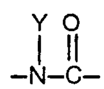

dans lequel Y est H en un alcoyle $C_1$—$C_3$, le groupe d'activation polaire étant juxtaposé au groupe non saturé.

5. Dérivé de glucoside selon la revendication 4 ayant la structure

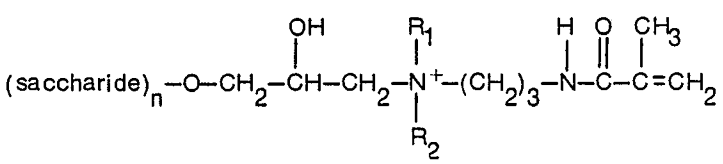 $M^-$

dans laquelle l'unité saccharide est le glucose, n est égal à 1 à 10 et $R_2$ est H ou un alcoyle $C_1$—$C_6$—.

6. Procédé pour préparer le dérivé de glucoside cationique de la revendication 1, comprenant les stades suivants:

a) faire réagir dans des conditions alcalines au moins une mole d'un glucoside réactif ayant la formule:

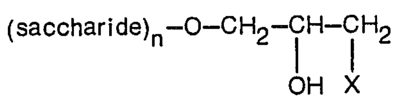 ou 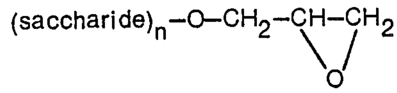

dans laquelle X est le chlore ou le brome, avec une mole d'une amine non saturée ayant la formule:

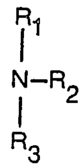 ou 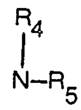

et

b) en isolant le dérivé de glucoside cationique obtenu.

7. Polymère du dérivé de glucoside cationique de la revendication 1, dans lequel le polymère est un homopolymère ou un copolymère avec au moins un autre comonomère non saturé.

8. Polymère selon la revendication 7, dans lequel:

$R_1$ est H ou un alcoyle $C_1$—$C_6$; $R_2$ est H, un alcoyle $C_1$—$C_6$, un alcényle ou un alcynyle $C_2$—$C_6$, ou A; $R_3$ est un alcényle ou un alcynyle $C_2$—$C_7$ ou est représenté par la formule —Z—C[Y]=$CH_2$ ou —Z—C≡CH dans laquelle Z est un groupe organique bivalent contenant un groupe d'activation polaire, juxtaposé au groupe non saturé et choisi parmi le groupe comprenant

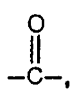, 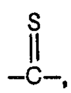, 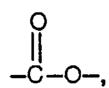, 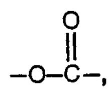, et 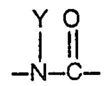

ou dans lequel:

$R_4$ et $R_5$ ensemble avec l'atome d'azote auquel ils sont liés, forment un hétérocycle non saturé à 5 ou 6 éléments substitués par un groupe représenté par la formule —C[Y]=$CH_2$ ou —C≡CH.

9. Polymère selon la revendication 8, dans lequel le glucoside monomère cationique à la structure:

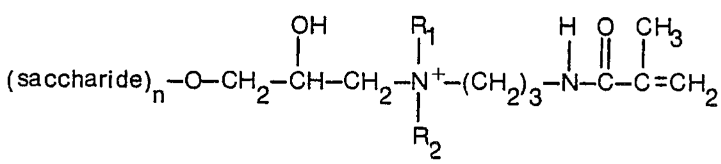 $M^-$

dans laquelle $R_2$ est H ou un alcoyle $C_1$—$C_6$.

10. Polymère selon la revendication 7, dans lequel le comonomère non saturé est choisi dans le groupe comprenant l'acrylamide, le méthacrylamide, l'acide acrylique et l'acide méthacrylique.